# EUROPEAN PATENT SPECIFICATION

(11) **EP 1 585 531 B1**
(45) Date of publication and mention of the grant of the patent: **11.09.2019**
(21) Application number: 03799967.9
(22) Date of filing: 18.12.2003
(51) Int. Cl.: A61K 31/718, A61M 1/28

(54) **BIOCOMPATIBLE DIALYSIS FLUIDS CONTAINING ICODEXTRINS**
BIOKOMPATIBLE, ICODEXTRINHALTIGE DIALYSIERFLÜSSIGKEITEN
LIQUIDES DE DIALYSE BIOCOMPATIBLES CONTENANT DES ICODEXTRINES

(30) Priority: 20.12.2002 US 327264
(43) Date of publication of application: 19.10.2005
(73) Proprietor: Baxter International Inc., Deerfield, IL 60015 (US); Baxter Healthcare S.A., 8152 Glattpark (Opfikon) (CH)
(72) Inventor: MARTIS, Leo, Long Grove, IL 60047 (US); CHOO, Carolyn, Long Grove, IL 60047 (US); ZIESKE, Paul, Glenview, IL 60025 (US)
(74) Representative: Alt, Michael
(86) International application number: PCT/US2003/040336
(87) International publication number: WO 2004/058277

(56) References cited:
- EP-A- 0 861 661
- EP-A- 1 354 607
- US-A- 5 092 838
- US-A- 5 536 469
- US-A- 5 827 820
- PEERS E ET AL: "ICODEXTRIN PROVIDES LONG DWELL PERITONEAL DIALYSIS AND MAINTENANCE OF INTRAPERITONEAL VOLUME" ARTIFICIAL ORGANS, BLACKWELL SCIENTIFIC PUBLICATIONS, INC., BOSTON, US, vol. 22, no. 1, 1998, pages 8-12, XP000945076 ISSN: 0160-564X
- MARTIS L ET AL: "PERITONEAL DIALYSIS SOLUTIONS FOR THE 21ST CENTURY" ARTIFICIAL ORGANS, BLACKWELL SCIENTIFIC PUBLICATIONS, INC., BOSTON, US, vol. 22, no. 1, 1998, pages 13-16, XP000901321 ISSN: 0160-564X
- Anonymous: "Extraneal Icodextrin 7.5% Peritoneal Dialysis Solution", , 4 June 2009 (2009-06-04), XP055530610, Retrieved from the Internet: URL:www.medsafe.govt.nz/Profs/datasheet/e/ Extranealsoln.htm [retrieved on 2018-12-05]

## Description

### BACKGROUND OF THE INVENTION

The present invention relates generally to multi chamber containers having two part peritoneal dialysis solutions.

Due to disease or insult or other causes, the renal system can fail. In renal failure of any cause, there are several physiological derangements. The balance of water, minerals (e.g., Na, K, Cl, Ca, P, Mg, SO₄) and the excretion of a daily metabolic load of fixed ions is no longer possible in renal failure. During renal failure, toxic end products of nitrogen metabolism (e.g., urea, creatinine, uric acid, and the like) can accumulate in blood and tissues.

Dialysis processes have been devised for the separation of elements in a solution by diffusion across a semi-permeable membrane (diffusive solute transport) across a concentration gradient. Examples of dialysis processes include hemodialysis, peritoneal dialysis and hemofiltration.

Hemodialysis treatment utilizes the patient's blood to remove waste, toxins, and excess water from the patient. The patient is connected to a hemodialysis machine and the patient's blood is pumped through the machine. Catheters are inserted into the patient's veins and arteries to connect the blood flow to and from the hemodialysis machine. Waste, toxins, and excess water are removed from the patient's blood and the blood is infused back into the patient. Hemodialysis treatments can last several hours and are generally performed in a treatment center about three or four times per week.

To overcome the disadvantages often associated with classical hemodialysis, other techniques were developed, such as hemofiltration and peritoneal dialysis. Hemofiltration is a convection-based blood cleansing technique. Blood access can be venovenous or arteriovenous. As blood flows through the hemofilter, a transmembrane pressure gradient between the blood compartment and the ultrafiltrate compartment causes plasma water to be filtered across the highly permeable membrane. As the water crosses the membrane, it convects small and large molecules across the membrane and thus cleanses the blood. An excessive amount of plasma water is eliminated by filtration. Therefore, in order to keep the body water balanced, fluid must be substituted continuously by a balanced electrolyte solution (replacement or substitution fluid) infused intravenously. This substitution fluid can be infused either into the arterial blood line leading to the hemofilter (predilution) or into the venous blood line leaving the hemofilter.

Peritoneal dialysis utilizes the patient's own peritoneum as a semipermeable membrane. The peritoneum is the membranous lining of the body cavity that, due to the large number of blood vessels and capillaries, is capable of acting as a natural semipermeable membrane.

In peritoneal dialysis, a sterile dialysis solution is introduced into the peritoneal cavity utilizing a catheter. After a sufficient period of time, an exchange of solutes between the dialysate and the blood is achieved. Fluid removal is achieved by providing a suitable osmotic gradient from the blood to the dialysate to permit water outflow from the blood. This allows a proper acid-base, electrolyte and fluid balance to be returned to the blood. The dialysis solution is simply drained from the body cavity through the catheter. Examples of different types of peritoneal dialysis include continuous ambulatory peritoneal dialysis, automated peritoneal dialysis and continuous flow peritoneal dialysis.

Standard peritoneal dialysis solutions contain dextrose at a concentration of 1.5% to 4.25% by weight to effect transport of water and metabolic waste products across the peritoneum. Although dextrose has the advantage of being relatively safe and inexpensive, it has a number of disadvantages. Because of the small size, dextrose is rapidly transported through the peritoneum, thus leading to the loss of osmotic gradient and loss of ultrafiltration within about 2 to 4 hours of infusion. It has been suggested that the ultrafiltration characteristics of peritoneal dialysis solutions could be improved by replacing dextrose with large molecular weight substances, such as icodextrin. Dialysis solutions containing icodextrin are commercially available and have been found to be useful in treating patients with end stage renal disease.

Like dextrose, glucose polymers are not stable during terminal heat sterilization (a pharmacoepial requirement for peritoneal dialysis fluids) if they are formulated at physiologic pH. As a result, icodextrin containing solutions are typically formulated at an acid pH, such as a pH between 5.0 to 5.5. However, the low pH can cause pain on infusion in some patients and is cytotoxic to peritoneal cells including mesothelial cells, macrophages and fibroblasts. In addition, even at pH 5.0 to 5.5, icodextrin can undergo degradation, thus resulting in a wide variety of degradation products that can lead to the formation of advanced glycation end products (AGEs). AGEs are believed to damage the peritoneal membrane.

EP-A-1,354,607 discloses a peritoneal dialysis solution comprising two separate solutions that are combined prior to administration. The first solution comprises a glucose polymer osmotic agent and the second solution comprises a buffer.

EP-A-0,861,661 discloses a peritoneal dialysis solution comprising maltodextrin.

US-5,827,820 discloses a peritoneal dialysis solution comprising two separate solutions that are combined prior to administration. The first solution comprises an osmotically active substance and the second solution comprises bicarbonate ions. The preferred osmotically active substance is glucose.

US-5,536,469 discloses a peritoneal dialysis solution comprising two separate solutions that are combined prior to administration. One of the solutions comprises glucose or a glucose-like compound, such as a glucose polymer.

US-5,092,838 discloses a peritoneal dialysis solution that is buffered with L-histidine. Two-part solutions having glucose or a glucose polymer osmotic agent that are mixed prior to use are mentioned.

Therefore, a need exists to provide improved medical solutions that can be readily manufactured, that can remain stable and sterile under storage conditions, and that can be readily and effectively used during medical therapy, such as dialysis therapy.

### SUMMARY OF THE INVENTION

According to the present invention, a multi chamber container having a two part peritoneal dialysis solution according to claim 1 is provided.

The dialysis solution is an icodextrin-based solution. The icodextrin based solution can be made at physiologic pH and with minimal glucose degradation products. This provides improved biocompatibility, particularly as applied during peritoneal dialysis.

The first solution is acidified with an acid, such as an organic acid (e.g., lactic acid, acetic acid, pyruvic acid and all of the intermediates of the KREBS tri-carboxylic acid cycle), an inorganic acid (e.g., hydrochloric acid), the like and combinations thereof. Further, the first solution includes about 100.0 to about 220.0 (g/L) of icodextrin and other components, such as calcium chloride, magnesium chloride, calcium chloride dihydrate, magnesium chloride hexahydrate, the like and combinations thereof. The buffer solution includes one or more components, such as sodium chloride, sodium lactate, sodium bicarbonate, the like and combinations thereof.

When mixed, the first part and the second part can form a mixed solution which includes, for example, about 4.0 to about 10.0 (g/dL) of icodextrin; about 0.5 to about 4.0 (mEq/L) of calcium; about 0.25 to about 2.0 (mEq/L) of magnesium; about 120.0 to about 135.0 (mEq/L) of sodium; about 90.0 to about 110.0 (mEq/L) of chloride; about 30.0 to about 45.0 (mEq/L) of lactate and the like. The mixed solution can further include, for example, about 5.0 mM or less of bicarbonate.

In an embodiment, the peritoneal dialysis solution of the present invention has a pH ranging from about 6.5 to about 7.4. A volume ratio of the first solution to the buffer solution can include about 3:1 to about 1:3.

In addition, a method of producing a peritoneal dialysis solution is described. The method includes preparing a first solution and a buffer solution wherein the first solution includes icodextrin, at a pH ranging from about 1.5 to about 5.0 and wherein the buffer solution has a pH ranging from about 7.0 to about 12.0; and mixing the first solution and the buffer solution prior to infusion into a patient.

In an embodiment, the peritoneal dialysis solution of the present invention has a first part consisting of a first solution containing icodextrin ranging from about 100 g/L to about 200 g/L, calcium, and magnesium wherein the first part has a pH ranging from about 2.5 to about 5.0; and a second part that includes sodium chloride and sodium lactate but does not contain amino acids with a pK¹ between 7 and 13, such as glycine, alanine and histidine, and has a pH of about 7 to about 12. The first part and the second part are so constructed and arranged that the first part and the second part are mixed to form a mixed solution prior to infusion into a patient wherein the mixed solution has a pH ranging from about 6.5 to about 7.4.

An advantage of the present invention is to provide improved peritoneal dialysis solutions.

Another advantage of the present invention is to provide peritoneal dialysis solutions which can be made at physiologic pH.

Furthermore, an advantage of the present invention is to provide peritoneal dialysis solutions with minimal glucose degradation products.

Moreover, an advantage of the present invention is to provide improved icodextrin-based solutions.

Another advantage of the present invention is to provide icodextrine-based solutions that can be effectively used during dialysis therapy, such as peritoneal dialysis.

Additional features and advantages of the present invention are described in, and will be apparent from, the following Detailed Description of the Invention and the figures.

### BRIEF DESCRIPTION OF THE FIGURES

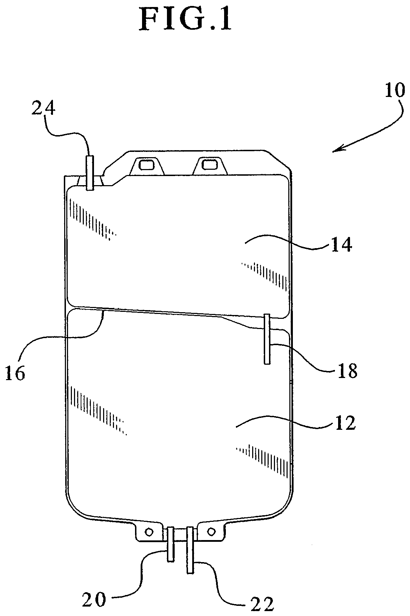

Fig.1 illustrates an icodextrin-based solution stored in a container pursuant to an embodiment of the present invention.

### DETAILED DESCRIPTION OF THE INVENTION

The present invention provides a multi chamber container housing a two part peritoneal dialysis solution. The dialysis solution is an icodextrin-based solution. As previously discussed, the icodextrin-based solution can be made at physiologic pH and with minimal glucose degradation products. This provides improved biocompatibility, particularly as applied during dialysis therapy, such as peritoneal dialysis.

With respect to dialysis therapy, the present invention can be used in a variety of different dialysis therapies to treat kidney failure. Dialysis therapy as the term or like terms are used throughout the text is meant to include and encompass any and all forms of therapies that utilize the patient's blood to remove waste, toxins and excess water from the patient. Such therapies, such as hemodialysis, hemofiltration and hemodiafiltration, include both intermittent therapies and continuous therapies used for continuous renal replacement therapy (CRRT). The continuous therapies include, for example, slow continuous ultrafiltration (SCUF), continuous venovenous hemofiltration (CWH), continuous venovenous hemodialysis (CVVHD), continuous venovenous hemodiafiltration (CVVHDF), continuous arteriovenous hemofiltration (CAVH), continuous arteriovenous hemodialysis (CAVHD), continuous arteriovenous hemodiafiltration (CAVHDF), continuous ultrafiltration periodic intermittent hemodialysis or the like. The icodextrin-based solution can be used during peritoneal dialysis including, for example, continuous ambulatory peritoneal dialysis, automated peritoneal dialysis, continuous flow peritoneal dialysis and the like. Further, although the present invention, in an embodiment, can be utilized in methods providing a dialysis therapy for patients having chronic kidney failure or disease, it should be appreciated that the present invention can be used for acute dialysis needs, for example, in an emergency room setting. Lastly, as one of skill in the art appreciates, the intermittent forms of therapy (i.e., peritoneal dialysis) may be used in the in center, self/limited care as well as the home settings.

The dialysis solution in the multiple chamber container can be used as a dialysate during any suitable dialysis therapy. Alternatively, the solutions can be administered or infused into a patient as a replacement solution, infusion solution or the like during dialysis therapy, particularly during continuous renal replacement therapy. In this regard, replacement solutions, infusion solutions or the like must necessarily be continuously fed to a patient as a substitute for an excessive amount of plasma water that is typically removed during continuous renal replacement therapy. In this regard, a proper water balance in the patient's body can be effectively maintained.

The icodextrin-based solution can include a variety of different components in any suitable amount. The solution at least includes two parts that are mixed prior to use. The first part consists of a first solution containing an icodextrin ranging from about 100.0 g/L to about 220.0 g/L and has a pH ranging from about 1.5 to about 5.0, such as 2.5, 3.0 and the like. In this regard, degradation of the icodextrin-based solution can be minimized during heat sterilization. It should be appreciated that the icodextrin-based solution can be sterilized in any suitable way, such as filtration sterilization, heat sterilization, steam sterilization, radiation sterilization and/or like sterilization techniques.

The first part can include a number of suitable and different types and amounts of components in addition to glucose polymer. For example, the first part includes an acid, such as an organic acid (e.g., lactic acid, acetic acid, pyruvatic acid and all of the intermediates of the KREBS tri-carboxylic acid cycle), an inorganic acid (e.g., hydrochloric acid), the like and combinations thereof. In an embodiment, the first solution includes about 100.0 to about 220.0 (g/L) of icodextrin, about 5.0 to about 10.0 (mEq/L) of calcium chloride dihydrate, about 0.5 to about 2.0 (mEq/L) of magnesium chloride hexahydrate, the like and combinations thereof.

The second part can include a variety of different and suitable materials. In an embodiment, the second part of the icodextrin-based solution includes a buffer solution at a pH ranging from about 7.0 to about 12.0 including, for example, sodium bicarbonate, sodium chloride, sodium lactate, the like and combinations thereof, but not one or more amino acids with a pK¹ between 7 and 13, such as histidine, glycine, alanine, etc., the like and combinations thereof.

It should be appreciated that the icodextrin-based solutions can include any suitable type, number and amount of additional components. For example, the solutions of the present invention can include one or more of any suitable type and amount of small molecular weight osmotic agents, such as glucose, glycerol, amino acids but no aminco acids with a pK¹ between 7 and 13 such as glycine, alanine and histidine, peptides, the like and combinations thereof. The small molecular weight osmotic agents of the first part can include, for example, glucose, glycerol and/or the like. In an embodiment, the small molecular weight osmotic agent concentration of the first part ranges from about 1% to about 6%. The small molecular weight osmotic agents of the second part can include, for example, amino acids but no aminco acids with a pK¹ between 7 and 13 such as glycine, alanine and histidine, peptides and/or the like. In an embodiment, the small molecular weight osmotic agent concentration of the second part ranges from about 1% to about 6%. When the first part and the second part are mixed and combined to form the icodextrin-based solution of the present invention, the small molecular weight osmotic agent concentration of the icodextrin-based solution, in an embodiment, ranges from about 0.5% to about 4%.

The pH can be adjusted to include any suitable pH within the pH range as discussed above. For example, the pH can be adjusted to about 7.0 to about 9.0, preferably to about 7.0 to about 8.0, using a pH stabilizer, such as sodium bicarbonate. In an embodiment, the pH of the buffer chamber can range from about 9.0 to about 12.0. This pH range can be effectively used when lactate is substituted with bicarbonate so that bicarbonate exists as carbonate. This would eliminate the need for a gas barrier overpouch to contain CO₂ within the solution.

The first part and the second part are so constructed and arranged that at least the first part and the second part are mixed prior to infusion into a patient. For example, the first part is stored in a first chamber of a multi-chamber container and the second part is stored in a second chamber of the multi-chamber container.

It should be appreciated that the components of the solution can be housed or contained in any suitable manner such that the icodextrin-based solutions can be effectively prepared and administered. Each part or component of the two part icodextrin-containing solution are formulated and stored separately, and then mixed just prior to use. A variety of containers can be used to house the two part glucose polymer-containing solution, such as separate containers (i.e., flasks or bags) that are connected by a suitable fluid communication mechanism. In an embodiment, a multi-chamber container or bag can be used to house the separate components of the solution as previously discussed. By way of further example, the solutions can be provided separately as concentrates and a mixing device, such as the BAXTER HOMECHOICE®, can be used to mix the solutions immediately prior to infusion.

Figure 1 illustrates a suitable container for storing, formulating and administering a bicarbonate-based solution of the present invention. The multi-chamber bag 10 has a first chamber 12 and a second chamber 14. The interior of the container is divided by a heat seal 16 into two chambers. It should be appreciated that the container can be divided into separate chambers by any suitable seal. In an embodiment, the container can be divided into separate chambers, such as two chambers, by a peel seal. The multi-chamber container 10 also has a frangible connector 18 to sealingly couple the first chamber 12 to the second chamber 14. To mix the solution within the multi-chamber bag 10, the frangible connector 18 is broken.

The first container or chamber 12 includes two port tubes having, for example, different lengths. As shown in Figure 1, the short port tube 20 can be utilized to add other constituents to the first chamber 12 during formulation of the solution of the present invention, if necessary. The long port tube 22 can be utilized to adaptedly couple the first chamber 12 to the patient via, for example, a patient's administration line (not shown). The second container or chamber 14 has a single port tube 24 extending therefrom which is closed by, for example, a solid rod (not shown). In this regard, it is not possible to add any additional constituents to this chamber and/or connect this chamber to a patient's administration line such that the chamber 14 cannot be adapted to deliver its constituents to the patient.

In an embodiment, the transfer of product within the multi-chamber bag 10 is thereby initiated from the second chamber 14 to the first chamber 12 such that the components of each chamber can be properly mixed to form the icodextrin-based solution. In this regard, the first chamber 12 is larger in volume than the second chamber 14 such that the components of each chamber can be properly mixed once the transfer from the second chamber to the first chamber has occurred. Thus, the multi-chamber bag 10 can house at least two solutions that after mixture will result in a ready-to-use dialysis solution. An example of the multi-chamber container is set forth in U.S. Patent No. 5,431,496. The multi-chamber bag can be made from a gas permeable material, such as polypropylene, polyvinyl chloride or the like.

In an embodiment, the container can be made with a gas barrier in any suitable way. For example, the gas barrier can be in the container material. Alternatively, the gas barrier can be an overpouch, a secondary liner or the like. The gas barrier can be composed of any suitable materials. In an embodiment, the gas barrier is composed of ethylvinyl acetate, polyvinyl dichloride, a copolymer of ethylvinyl acetate and polyvinyl dichloride, other suitable materials including polymeric materials and combinations thereof.

It should be appreciated that the container of the present invention can be manufactured from a variety of different and suitable materials and configured in a number of suitable ways such that the icodextrin-based solution can be effectively formulated and administered to the patient during medical therapy. For example, the second chamber can be larger in volume than the first chamber such that the icodextrin-based solution can be readily and effectively made and administered to the patient from the second chamber.

The icodextrin-based solution is prepared by mixing at least two parts prior to use. In an embodiment, the mixed icodextrin-based solution at least includes about 4.0 to about 10.0 (g/dL) of icodextrin, about 0.5 to about 4.0 (mEq/L) of calcium, about 0.25 to about 2.0 (mEq/L) of magnesium, about 120.0 to about 135.0 (mEq/L) of sodium, about 90.0 to about 110.0 (mEq/L) of chloride, about 30.0 to about 45.0 (mEq/L) of lactate, the like and combinations thereof. For example, the mixed solution can include about 5.0 mM or less of bicarbonate.

In an embodiment, the mixed solution has a pH ranging from about 6.5 to about 7.4. The pH stabilizer of the second part can be included in the mixed solution, in an embodiment, in an amount ranging from about 25.0 mEq/L to about 45.0 mEq/L. The icodextrin-based solution includes, in an embodiment, a volume ratio of the icodextrin-containing solution and the buffer solution that ranges from about 3:1 to about 1:3.

By way of example and not limitation examples of the present invention will now be set forth.

### COMPOSITION EXAMPLE ONE

**COMPOSITION IN GLUCOSE POLYMER CHAMBER**

| | |
|---|---|
| Icodextrin (g/L) | 100.0 - 220.0 |
| Calcium Chloride dihydrate (mEq/L) | 5.0 - 10.0 |
| Magnesium Chloride hexahydrate (mEq/L) | 0.5 - 2.0 |
| HCl for pH adjustment between 2.5 and 5.0 | |

**COMPOSITION OF THE BUFFER CHAMBER**

| | |
|---|---|
| Sodium Chloride (mEq/L) | 50.0 - 150.0 |
| Sodium Lactate (mEq/L) | 50.0 - 120.0 |
| Sodium Bicarbonate for pH adjustment between 8.0 and 9.0 | |

### COMPOSITION EXAMPLE TWO

**COMPOSITION IN GLUCOSE POLYMER CHAMBER (Large Chamber)**

| | |
|---|---|
| Icodextrin (g/L) | 121 |
| Sodium Chloride (g/L) | 4.22 |
| Calcium Chloride Dihydrate (g/L) | 0.40 |
| Magnesium Chloride Hexahydrate (g/L) | 0.08 |
| Sodium Lactate (g/L) | 3.50 |
| pH | about 5.0 to about 5.4 |

**COMPOSITION IN BUFFER CHAMBER (Small Chamber)**

| | |
|---|---|
| Sodium Chloride (g/L) | 7.42 |
| Sodium Lactate (g/L) | 6.15 |
| Sodium Bicarbonate (g/L) | 0.58 |
| pH | about 8.2 to about 8.7 |

**ICODEXTRIN AND IONIC COMPOSITION OF THE MIXED SOLUTION**

| | |
|---|---|
| Icodextrin (g/dL) | 4.0 - 10.0 |
| Calcium (mEq/L) | 0.5 - 4.0 |
| Magnesium (mEq/L) | 0.25 - 2.0 |
| Sodium (mEq/L) | 120.0 - 135.0 |
| Chloride (mEq/L) | 90.0 - 110.0 |
| Lactate (mEq/L) | 30.0 - 45.0 |
| Bicarbonate (mM) | NMT 5.0 |

| | |
|---|---|
| As used herein, the term "NMT" means not more than. | |

**ICODEXTRIN CHARACTERISTICS**

| | |
|---|---|
| Weight Average Molecular Weight | 10,000 - 20,000 |
| Number Average Molecular weight | 4,000 - 8,000 |
| Polydispersity | 1.0 - 4.0 |
| Fraction > 100,000 | NMT 1.0% |
| Mono, Di, Tri- Saccharides | NMT 5.0% |
| Linear Polymers (alpha 1,4) | NLT 90.0% |
| Branched Polymers (alpha 1,6) | NMT 10.0% |
| Aluminum (10% solution) | <10 ppb |
| Aqueous Solubility | NLT 22.0% |
| Heavy Metals | <5 ppm |

| | |
|---|---|
| As used herein, the term "NLT" means not less than. | |

**DEGREE OF POLYMERIZATION OF ICODEXTRIN (DP)**

| | |
|---|---|
| DP greater than 20 | >75% |
| DP greater than 40 | >50% |
| DP greater than 80 | >25% |

### EXPERIMENT ONE

This experiment was performed to determine the effect of pH on the stability of icodextrin (7.5% solution). Stability of icodextrin was assessed by measuring the absorbency of icodextrin solutions at different pH values before and after sterilization:

| Pre-sterilization (ph) | Post-sterilization (ph) | AU 284 nm | AU 228 nm |
|---|---|---|---|
| 5.5* | 5.4 | 0.022 | 0.044 |
| 4.0 | 3.9 | 0.011 | 0.012 |
| 3.5 | 3.5 | 0.013 | 0.010 |
| 3.0 | 3.0 | 0.011 | 0.010 |
| 2.5 | 2.5 | 0.016 | 0.014 |

| | | | |
|---|---|---|---|
| *This was a commercially available icodextrin solution. The remaining solutions tested pursuant to EXPERIMENT ONE were prepared according to an embodiment of the present invention. | | | |

The data of EXPERIMENT ONE suggest that the degradation of icodextrin could be reduced by more than 50% by adjusting pre-sterilization pH between 2.5 and 4.0. It is noted that too acidic of a pH results in hydrolysis of icodextrin that results in a change of the molecular weight of the icodextrin. The optimum pH of the icodextrin chamber is where hydrolysis and degradation are minimal.

### EXPERIMENT TWO

This experiment was performed to determine the pH of the mixed solution that was prepared according to an embodiment of the present invention.

Part One solution was prepared by mixing the following components in 1 liter of solution:

| | |
|---|---|
| Icodextrin | 207 gms |
| Calcium chloride dehydrate | 0.710 gms |
| Magnesium chloride hexahydrate | 0.140 gms |
| HCl added to adjust the pH to 3.0 | |
| Solution volume | 758 mL |

Part Two solution was prepared by mixing following components in 1 liter of solution:

| | |
|---|---|
| Sodium chloride | 8.44 gms |
| Sodium lactate | 7.03 gms |
| Sodium bicarbonate added to adjust the pH to 8.3 | |
| Solution volume | 1332 ml |

The Part One and Part Two solutions were combined to form a mixed solution with the following composition:

| | |
|---|---|
| Icodextrin | 7.5 gm/dL |
| Calcium | 3.5 mEq/L |
| Magnesium | 0.5 mEq/L |
| Sodium | 132 mEq/L |
| Chloride | 96 mEq/L |
| Lactate | 40 mEq/L |
| pH | 7.0 |

The results of EXPERIMENT TWO indicate that the two part solution prepared as discussed above pursuant to an embodiment of the present invention has a composition that is ideal for use in peritoneal dialysis. The two part solution and the use of pH adjustor in a manner described above pursuant to an embodiment of the present invention provides icodextrin-based solutions that can be prepared with improved stability, pH and thus enhanced biocompatibility.

It should be understood that various changes and modifications to the presently preferred embodiments described herein will be apparent to those skilled in the art.

## Claims

1. A multiple chamber container housing a two part peritoneal dialysis solution wherein
the first part is stored in a first chamber of the multiple chamber container and wherein the first part consists of a first solution containing icodextrin ranging from about 100.0 g/L to about 220.0 g/L, wherein the first part has a pH ranging from about 1.5 to about 5.0; and wherein
the second part is stored in a second chamber of the multiple chamber container and wherein
the second part comprises a buffer solution having a pH ranging from about 7.0 to about 12; and wherein the buffer solution does not contain amino acids with a pK¹ between 7 and 13, such as glycine, alanine and histidine; and wherein the first part and the second part being so constructed and arranged that the first part and the second part are mixed prior to infusion into a patient.

2. The multiple chamber container as claimed in claim 1, wherein the buffer solution comprises sodium lactate.

3. The multiple chamber container as claimed in claim 1, wherein the buffer solution comprises sodium bicarbonate.

4. The multiple chamber container as claimed in claim 1, wherein the first part and the second part when combined form a mixed solution which includes:
about 4.0 to about 10.0 (g/dL) of icodextrin;
about 0.5 to about 4.0 (mEq/L) of calcium;
about 0.25 to about 2.0 (mEq/L) of magnesium;
about 120.0 to about 135.0 (mEq/L) of sodium;
about 90.0 to about 110.0 (mEq/L) of chloride;
about 30.0 to about 45.0 (mEq/L) of lactate,
and 5 mM or less of bicarbonate.

5. The multiple chamber container as claimed in claim 1, wherein the first solution comprises lactic acid.

6. The multiple chamber container as claimed in claim 1, wherein the first solution comprises an inorganic acid.

## Patentansprüche

1. Mehrkammerbehälter, der eine zweiteilige Peritonealdialyselösung enthält, wobei
der erste Teil in einer ersten Kammer des Mehrkammerbehälters gelagert ist und wobei der erste Teil aus einer ersten Lösung besteht, die etwa 100,0 g/l bis etwa 220,0 g/l Icodextrin enthält, wobei der erste Teil einen pH-Wert von etwa 1,5 bis etwa 5,0 aufweist; und wobei
der zweite Teil in einer zweiten Kammer des Mehrkammerbehälters gelagert ist und wobei der zweite Teil eine Pufferlösung mit einem pH-Wert von etwa 7,0 bis etwa 12 umfasst; und wobei
die Pufferlösung keine Aminosäuren mit einem pK¹-Wert zwischen 7 und 13, wie z. B. Glycin, Alanin und Histidin, enthält; und wobei
der erste Teil und der zweite Teil so aufgebaut und angeordnet sind, dass der erste Teil und der zweite Teil vor der Infusion in einen Patienten gemischt werden.

2. Mehrkammerbehälter gemäß Anspruch 1, wobei die Pufferlösung Natriumlactat umfasst.

3. Mehrkammerbehälter gemäß Anspruch 1, wobei die Pufferlösung Natriumbicarbonat umfasst.

4. Mehrkammerbehälter gemäß Anspruch 1, wobei der erste Teil und der zweite Teil, gemeinsam eine gemischte Lösung bilden, die enthält:
etwa 4,0 bis etwa 10,0 (g/dl) Icodextrin;
etwa 0,5 bis etwa 4,0 (meq/l) Calcium;
etwa 0,25 bis etwa 2,0 (meq/l) Magnesium;
etwa 120,0 bis etwa 135,0 (meq/l) Natrium;
etwa 90,0 bis etwa 110,0 (meq/l) Chlorid;
etwa 30,0 bis etwa 45,0 (meq/l) Lactat;
und 5 mM oder weniger Bicarbonat.

5. Mehrkammerbehälter gemäß Anspruch 1, wobei die erste Lösung Milchsäure umfasst.

6. Mehrkammerbehälter gemäß Anspruch 1, wobei die erste Lösung eine anorganische Säure umfasst.

## Revendications

1. Récipient à compartiments multiples renfermant une solution de dialyse péritonéale en deux parties,
la première partie étant stockée dans un premier compartiment du récipient à compartiments multiples et la première partie étant constituée d'une première solution contenant de l'icodextrine dans la plage d'environ 100,0 g/L jusqu'à environ 220,0 g/L, la première partie possédant un pH dans la plage d'environ 1,5 jusqu'à environ 5,0 ; et
la deuxième partie étant stockée dans un deuxième compartiment du récipient à compartiments multiples et la deuxième partie comprenant une solution tampon possédant un pH dans la plage d'environ 7,0 jusqu'à environ 12 ;
et la solution tampon ne contenant pas d'acides aminés présentant un pK¹ entre 7 et 13, tel que la glycine, l'alanine et l'histidine ; et
la première partie et la deuxième partie étant conçues et disposées de telle façon que la première partie et la deuxième partie sont mélangées avant la perfusion au patient.

2. Récipient à compartiments multiples selon la revendication 1, la solution tampon comprenant du lactate de sodium.

3. Récipient à compartiments multiples selon la revendication 1, la solution tampon comprenant du bicarbonate de sodium.

4. Récipient à compartiments multiples selon la revendication 1, la première partie et la deuxième partie, lorsqu'elles sont combinées, formant une solution mixte qui comporte :
environ 4,0 jusqu'à environ 10,0 (g/dL) d'icodextrine ;
environ 0,5 jusqu'à environ 4,0 (mEq/L) de calcium ;
environ 0,25 jusqu'à environ 2,0 (mEq/L) de magnésium ;
environ 120,0 jusqu'à environ 135,0 (mEq/L) de sodium ;
environ 90,0 jusqu'à environ 110,0 (mEq/L) de chlorure ;
environ 30,0 jusqu'à environ 45,0 (mEq/L) de lactate
et 5 mM ou moins de bicarbonate.

5. Récipient à compartiments multiples selon la revendication 1, la première solution comprenant de l'acide lactique.

6. Récipient à compartiments multiples selon la revendication 1, la première solution comprenant un acide inorganique.
